# EUROPEAN PATENT SPECIFICATION

(11) **EP 2 356 457 B1**
(45) Date of publication and mention of the grant of the patent: **16.05.2018**
(21) Application number: 08875824.8
(22) Date of filing: 13.10.2008
(51) Int. Cl.: G01N 33/543

(54) **METHOD FOR THE PREPARATION OF IMMUNOCONJUGATES AND USE THEREOF**
VERFAHREN ZUR HERSTELLUNG VON IMMUNOKONJUGATEN UND VERWENDUNG DAVON
PROCÉDÉ POUR LA PRÉPARATION D'IMMUNOCONJUGUÉS ET UTILISATION DE CEUX-CI

(43) Date of publication of application: 17.08.2011
(73) Proprietor: Xeptagen SPA, 30175 Venezia Marghera (IT)
(72) Inventor: PENGO, Paolo, I-30175 Venezia Marghera (IT); BENEDUCE, Luca, I-30175 Venezia Marghera (IT); FASSINA, Giorgio, I-30175 Venezia Marghera (IT)
(74) Representative: Vinci, Marcello
(86) International application number: PCT/IB2008/054203
(87) International publication number: WO 2010/043927

(56) References cited:
- WO-A1-2005/003771
- WO-A2-2005/050224
- US-A1- 2004 166 505
- FRENGEN J ET AL: "Dual analyte assay based on particle types of different size measured by flow cytometry" JOURNAL OF IMMUNOLOGICAL METHODS, ELSEVIER SCIENCE PUBLISHERS B.V.,AMSTERDAM, NL, vol. 178, no. 1, 13 January 1995 (1995-01-13), pages 141-151, XP004021133 ISSN: 0022-1759
- WANG C ET AL: "Synthesis of aqueous CdTe quantum dots embedded silica nanoparticles and their applications as fluorescence probes" TALANTA, ELSEVIER, AMSTERDAM, NL, vol. 77, no. 4, 19 September 2008 (2008-09-19), pages 1358-1364, XP025780965 ISSN: 0039-9140 [retrieved on 2008-09-19]
- BENEDUCE ET AL: "Detection of prostate-specific antigen coupled to immunoglobulin M in prostate cancer patients" CANCER DETECTION AND PREVENTION, ELSEVIER SCIENCE, vol. 31, no. 5, 26 November 2007 (2007-11-26), pages 402-407, XP022370706 ISSN: 0361-090X
- Luca Beneduce ET AL: "Squamous cell carcinoma antigen-immunoglobulin M complexes as novel biomarkers for hepatocellular carcinoma", CANCER., vol. 103, no. 12, 1 January 2005 (2005-01-01), pages 2558-2565, XP055336217, US ISSN: 0008-543X, DOI: 10.1002/cncr.21106
- LAITINEN ET AL: "Brave new (strept)avidins in biotechnology", TRENDS IN BIOTECHNOLOGY, ELSEVIER PUBLICATIONS, CAMBRIDGE, GB, vol. 25, no. 6, 17 May 2007 (2007-05-17), pages 269-277, XP022081816, ISSN: 0167-7799, DOI: 10.1016/J.TIBTECH.2007.04.001

## Description

The present patent describes a method for the synthesis of molecular species and namely a method for the synthesis of conjugates and a new method to optimize the reactivity of said conjugates and the use thereof in procedures pertinent to oncology diagnostics. Tumors represent nowadays the second leading cause of death after cardiovascular diseases and their incidence rate is increasing worldwide. Although the distribution and the incidence of each specific type of cancer are very different when different world regions are concerned, said incidence rates show very similar trends in industrialized countries. In these countries, the neoplastic diseases of the lungs and prostate gland represent a large part of the reported cancer cases within the male population. Within the female population a large part of the reported cancer cases are breast cancer, while the incidence of lung cancer is increasing. Besides these gender-specific tumors, the incidence rates of intestinal cancer and pancreatic carcinoma are continuously increasing, in facts, they doubled in the last 50 years. From these considerations clearly stems the importance of the quest for specific markers able to signal the neoplastic transformation with high organ-specificity. The presence of tumors often produces a specific molecular signature as the result of the altered metabolism in those cells that underwent neoplastic transformation. The presence of these substances, known as tumor biomarkers or tumor associated antigens, may be detected exploiting histological methods or, more conveniently from the clinical point of view, by means of analyses carried out on biological fluids such as serum or plasma. However, in some cases, the modification of the normal biomarker profile may not be an useful index of the presence of neoplastic diseases. This occurs because these modifications may be the result of lifestyle, non neoplastic diseases or to be iatrogenic (Gion, M. in : "Guida all'utilizzo dei biomarcatori in oncologia" Biomedia Source Books edition, Milan 2002, ITALY). From the diagnostic point of view, this is a source of false-positive results within a cohort of healthy individuals.

The same applicant owns a patent application concerning an highly specific method for the diagnosis of cancer, where the identification of the biomarker does not give indication of a pathologic condition in healthy subjects. In closer details: the applicant devised tests capable to attain a better correlation between the presence of a biomarker and the pathologic condition of the subjects. These tests have been developed from the experimental evidence that in sera of patients affected by neoplastic diseases, besides the presence of free biomarkers, i.e. biomarkers not conjugated to other co-factors, other biomarker containing species may be detected. The latter species are complexes of said biomarkers with immunoglobulins, namely immunoglobulins of the M class or IgM. Hence, a method based on the identification of the circulating immunocomplexes formed by the association of tumor marker and autoantibody specific thereto, hereinafter referred to as specific CIC, has been conceived. This method is extremely useful in cancer diagnosis, since it allows the discrimination of healthy subjects from a cohort of patients affected by cancer with higher selectivity with respect to tests based on the detection of the free tumor antigen. Autoantibodies are antibodies naturally produced by immune system and directed against an antigen recognized as non-self, even though it is one of the molecules normally expressed by the organism. The common explanation for the immunogenicity of these self species is related to their anomalously high expression (hyperexpression) in tissues that underwent neoplastic transformation when compared to normal tissues (Sahin U. et al. Proc. Natl. Acad. Sci. USA, 1995, 92, 11810-13). For the reasons outlined above, the analysis of the free tumor antigens may have poor diagnostic value, especially when high specificities are concerned. Instead, these considerations do not apply to the immunocomplexes analysis which allows the discrimination of healthy subjects from cancer patients with enhanced specificity. The effects of the IC formation on the determination of the free tumor-associated antigens in serum or plasma are twofold: (i) When the antigen is involved in the CIC, it may be masked and thus not detected with a normal immunometric assay, this condition may explain the occurrence of false negative results. On the other hand, low levels of antigen released from normal cells may not form stable and persistent ICs. In the latter case, the free antigen may be detected leading to false positive results. Both sources of false results may be eliminated revealing the specific CIC as it is described in one embodiment of the following invention.

In different cancers, including HCC, it is possible to detect serological immunoglobulin-M (IgM) against tumor markers; these complexes represent the immune system response to tumor antigens and/or to cancer cells [Volmmers HP, et al. The "early birds": natural IgM antibodies and immune surveillance. Histol Histopathol. 20:927-37. 2005]

Detection of SCCA-IgM complexes has been found to be efficient to identify liver damage. Levels of SCCA-IgM in HCC patients were higher when compared to patients with cirrhosis and chronic hepatitis [Beneduce L. et al. Squamous cell carcinoma antigen-immunoglobulin M complexes as novel biomarkers for hepatocellular carcinoma. Cancer. 103:2558-65.2005]

The diagnostic method devised by the applicant allows the simple determination of the tumor associated biomarker by means of specific reagents.

The research activity developed, on a large number of neoplastic diseases for which an antigenic biomarker is already known, led to the detection of immunocomplexes formed by said antigenic biomarker and autoantibody specific thereto. The detection of said specific complex is useful in the diagnosis for the given tumor. Said method for cancer diagnosis is based on the detection, in biological fluids, of specific CIC by contacting and incubating the samples obtained from the subject with specific reagents (SR) directed against the tumor associated antigen. Said method comprises the detection of the SR-CIC complex, where the presence of said complex indicates the presence of cancer. The SR considered comprise polyclonal and/or monoclonal antibodies or fragments thereof to be used to reveal, by immunometric methods, the presence of tumor markers involved in the formation of specific CIC and present in the blood of patients affected by neoplastic diseases. Non-limiting examples of said immunometric methods used to assess the CIC content in biological fluids comprise: direct, indirect or "sandwich" ELISAs (enzyme linked immunosorbent assays), radiometric assays (RIAs) or "sandwich" radiometric assays (IRMAs), or homogeneous immunometric assays exploiting different technologies such as "rapid-near-infrared spectroscopy".

A typical example of immunoenzymatic methods for the detection in biological fluids of the specific CIC comprises:
1] Immobilization, on plastic microtiter plates for ELISA determinations or on particles to be used for diagnostic purposes, of specific reagents (SR) directed against the tumor marker in either the free or conjugated form;
2] Incubation of the biological fluid obtained from the patients with the immobilized compound;
3] Washing of the complex formed by the immobilized compound and the IC;
4] Incubation of said complex with polyclonal or monoclonal antibodies directed against antibodies of human origin, conjugated to traceable markers such as enzymes; chromogenic, fluorescent, radioactive, chemiluminescent compounds, coenzymes, or enzyme inhibitors;
5] Removal of the excess of antibody by suitable washing steps.
6] Detection of the adsorbed antibody by suitable reagents.

As used herein, biological fluids means: serum, plasma, lymphatic fluid, ascites, pleuric exudates, spinal fluid etc obtained from patients by conventional methods in amounts sufficient for performing assays thereon. These techniques are described in the literature and known by those skilled in the art.

Immunometric methods heretofore known are relative by nature. They do no allow the absolute determination of the concentration of an analyte of interest, but they allow the comparison of a signal proportional to said concentration with a similar signal obtained by using one or more samples of known concentration as calibrators. The immunometric methods of quantitative analysis cannot be devoided of calibration methodologies. In the clinical practice, and in diagnostic procedures, the analytes of interest are compounds the concentration thereof or their variation, are associated to the onset or the evolution of pathologic conditions. Among these compounds, there are species correlated to the development of neoplastic diseases, referred to as tumor markers or tumor associated antigens. Standard preparations of said compounds require their isolation from natural sources, and among them, biological material obtained from subjects affected by neoplastic diseases; alternatively, these species can be obtained exploiting recombinant DNA technologies. In the first case, the preparation of said calibrators requires handling and processing material of human origin, which poses serious supplying limitations and safety issues in the implementation of efficient industrial processes. In the second case the availability of recombinant products solve any supplying and safety problems, however, the expression of recombinant proteins is feasible only for molecular species of well defined composition and known sequence. There are however, analytes with relevance in the diagnosis of neoplastic diseases such as the aforementioned biomarker-autoantibody immunocomplexes, that are intrinsically heterogeneous and for them the expression as recombinant products is not practicable. At the present stage, the generation of calibrators for the quantitative analysis of these compounds, is possible only through extraction processes from samples of human origin. The introduction of synthetic or semi-synthetic processes for the obtainment of molecular species to be used as substitutes of said calibrators obtained by extraction from samples of human origin would simplify the productive processes increasing their safety standards reducing at the same time their costs and ensuring a better lot to lot reproducibility. A first object of the present invention is a method for the synthesis of molecular species, hereinafter referred to as conjugates, displaying the reactivity of the biomarker-IgM immunocomplexes. The method of the present invention overcome all the problems related to the obtainment of biomarker-IgM immunocomplexes to be used as calibrators in diagnostic kits for cancer detection.

In Table 1, we report as a non-limiting example, biomarker and neoplasias of interest of the new method.

**TABLE 1**

| ***Tumor Marker*** | ***Cancer*** | ***Reference*** |
|---|---|---|
| Ki-67 | Astrocytoma | Br J Cancer 89(1):128-34, 2003 |
| Fibronectin | Bladder cancer | Clin Chem Lab Med 41(8):1069-74,2003 |
| Hepatoma up regulated protein (HURP) | Bladder cancer | Anticancer Res 23(3B):2729-33, 2003 |
| Mucin 7 (MUC7) | Bladder cancer | Urology 62(1):182-86, 2003 |
| NMP22 | Bladder cancer | J Chin Med Assoc 66(5):294-98, 2003 |
| NMP22 | Bladder cancer | Anticancer Res 23(2A):805-12, 2003 |
| Prostate stem cell antigen (PSCA) | Bladder cancer | J Urol 169(6):2094-100, 2003 |
| Telomerase | Bladder cancer | Urology 62(2):362-67, 2003 |
| Tissue polypeptide antigen (TPA) | Bladder cancer | Urology 62(2):243-48, 2003 |
| CA 15-3, CA 27.29, AFP, CEA | Breast cancer | Biomed Sci Instrum 39:408-14, 2003 |
| CA 15-3, Ceruloplasmin, TPA | Breast cancer | East Afr Med J 77(6):291-94, 2000 |
| CEA, Cytokeratin 19 (CK19), Maspin | Breast cancer | Anticancer Res 23(2C):1883-90, 2003 |
| c-Met | Breast cancer | Breast Cancer Res 5(3):71-76, 2003 |
| Cytochrome P450 3A4 | Breast cancer | Biomed Sci Instrum 39:24-29, 2003 |
| Epithelial glycoprotein 2 (EGP2), Cytokeratin 19 (CK19) | Breast cancer | Int J Cancer 106(4):611-18, 2003 |
| MUC 1 | Breast cancer | Int J Biol Marker 15(4): 343-56, 2000 |
| Her2/Neu | Breast cancer | Clin Chem 49(10): 1579-98, 2003 |
| MMP-9 | Breast cancer | Int J Cancer 106(5):745-51, 2003 |
| Human kallikrein 5 (hK5) | Breast cancer and ovarian cance | Cancer Res 63(14):3958-65, 2003 |
| NMP179 | Cervical squamous epithelium c | Anticancer Res 23(2A):805-12, 2003 |
| CEA, Cytokeratin 19 (CK19), Cytokeratin 20 (CK20) | Colorectal cancer | J Cancer Res Clin Oncol 129(3): 192-98, 2003 |
| Cytokeratin | Colorectal cancer | Colorectal Dis 5(2):164-68, 2003 |
| Cytokeratin 20 (CK20), CEA, Guanylyl cyclase C (GCC) | Colorectal cancer | Eur J Cancer 39(9):1234-41, 2003 |
| Her2/Neu | Colorectal cancer | Int J Cancer 105(6):796-802, 2003 |
| MUC6, MUC5AC | Colorectal cancer | Glycocong J 18(11-12):907-14, 2001 |
| RelA, NF-kb | Colorectal cancer | Oncology 65(1):37-45, 2003 |
| Bcl-6, CD10 | B-cells lymphoma | Human Pathol 34(6):610-16, 2003 |
| CEA | Endometrial carcinoma | Anticancer Res 23(2A):1103-06, 2003 |
| Cystein-rich fibroblast growth factor receptor 1 (CFR-1) | Stomach cancer | Cancer Res 63(9):2052-61, 2003 |
| p27, MIB-1 | Stomach cancer | Pathologica 95(1):22-30, 2003 |
| Chromogranin A (CgA), Neuron specific enolase (NSE), Synaptophysin, Leu-7, beta III-tubulin | Gastrointestinal carcinoma | Cesk Pathol 39(2):47-53, 2003 |
| Cytokeratin, Epithelial membrane antigen (EMA) | Head and neck carcinoma | Laryngoscope 113(5):892-96, 2003 |
| Hyaluronidase (HYAL1) | Head and neck carcinoma | Int J Cancer 106(3):438-45, 2003 |
| Latent membrane protein 1 (LMP-1) | Head and neck carcinoma | Cancer 97(8):1909-13, 2003 |
| Alpha-fetoprotein (AFP), Des-gamma-carboxy prothrombin (DCP) | Hepatocellular carcinoma | Eur J Gastroenterol Hepatol 15(6):641-48 |
| Cellular retinol binding protein 1 (CRBP1) | Hepatocellular carcinoma | Hepatology 38(2):470-80, 2003 |
| Glypcan-3 | Hepatocellular carcinoma | Gastroenterology 125(1):89-97, 2003 |
| Telomerase | Hepatocellular carcinoma | Oncology 64(4):430-34, 2003 |
| Human cervical cancer oncogene (HCCR) | Hepatocellular carcinoma | Cancer Res 64, 5434-41, 2004 |
| Osteopontin (OPN) | Hepatocellular carcinoma | Am J Gastroenterol 101: 2051-59, 2006 |
| Vascular endothelial growth factor (VEGF) | Hepatocellular carcinoma | J Gastroenterol 33(3): 376-82, 1998 |
| Tissue inhibitor of metalloproteinases 1 (TIMP 1) | Hepatocellular carcinoma | Liver Int 24(4): 379-83, 2004 |
| Survivin | Hepatocellular carcinoma | World J Gastroenterol 13(46):6264-8, 2007 |
| Heat shock protein 27 (HSP 27) | Hepatocellular carcinoma | Cancer 88(11): 2464-70, 2000 |
| Clusterin | Hepatocellular carcinoma | Human Pathol 35(11): 1340-6, 2004 |
| Preferentially expressed antigen of melanoma (PRAME) | Leukemia, Multiple myeloma | Leuk Lymphoma 44(3):439-44, 2003 |
| CEA, Chromogranin A, NSE, Vascular endothelial growth factor (VEGF), Stem cell factor (SCF), Hepatocyte growth factor/Scatter factor (HGF/SF) | Lung cancer | Anticancer Res 23(1A):49-62, 2003 |
| Epidermal growth factor receptor (EGFR) | Lung cancer | Int J Clin Oncol 8(2):79-82, 2003 |
| M2-PK, CYFRA 21-1, NSE, SCC | Lung cancer | Anticancer Res 23(2A):899-906, 2003 |
| Neuron specific enolase (NSE), S-100B, Tyrosinase, LDH | Melanoma | Magy Onkol 47(1):89-104, 2003 |
| Cytokeratin 20 (CK20), Prostate stem cell antigen (PSCA) | Gastrointestinal cancer | Anticancer Res 23(3B):2711-16, 2003 |
| Medkine (MK) | Gastric carcinoma | BBRC 306(2):329-32, 200 |
| CA 125 | Ovarian cancer | Oncology 65(1):1-6, 2003 |
| CASA, CA 125 | Ovarian cancer | Anticancer Res 23(2A):1115-18, 2003 |
| HE4 | Ovarian cancer | Adv Exp Med Biol 622: 15-21, 2008 |
| Mesothelin | Ovarian cancer | Gynecol Oncol 99: 267-77, 2005 |
| CA 19-9 | Pancreatic carcinoma | Anticancer Res 23(2A):835-40, 2003 |
| Survivin, p53, Bcl-2 | Pancreatic carcinoma | Int J Gastrointest Cancer 32(2-3):73-81, 2002 |
| Secretogranin II-derived peptide EM66 | Pheochromocytoma | J Clin Endocrinol Metab 88:2579-85, 2003 |
| GLUT1, GLUT12 | Prostate cancer | Cancer 97(8):2035-42, 2003 |
| Insulin-like growth factor binding protein 2 (IGBFB2) | Prostate cancer | Virchows Arch 442(4):329-35, 2003 |
| Prostate-specific antigen (PSA) | Prostate cancer | Ann Clin Biochem 38: 633-51, 2001 |
| PSMA prostate-specific membrane antigen (PSMA) | Prostate cancer | Rev Urol 6 Suppl 10: S13-8 |
| alpha-methylacyl-CoA racemase (AMACR) | Prostate cancer | JAMA 287:1662-1670, 2002 |
| Myogenin, MyoD1 | Rhabdomyosarcoma | J Clin Pathol 56(6):412-16, 2003 |
| Dipeptidyl Peptidase IV (DPP IV/CD 26) | thyroid cancer (papillary carcinoma) | Neoplasma 50(3):159-64, 2003 |
| Peroxisome proliferating activated receptor gamma (PPAR gamma) | thyroid cancer (papillary carcinoma) | Am J Clin Pathol 120(2):175-81,2003 |

An embodiment of the present invention is a method useful in the determination of concentration ratios or mass ratios, in either an explicit or implicit form, as function of parameters, between at least two different biomolecules, functionalized or unfunctionalized fragments, functionalized or unfunctionalized sequences thereof (hereinafter referred to as non accessory components) involved in the presence or absence of at least a third molecule or biomolecule acting as a crosslinker or scaffold (hereinafter referred to as accessory component) in a single polydispersed or monodispersed molecular construct, hereinafter referred to as conjugate, with general formula AₐB_{b}C_{c}.....Z_{z} endowed with the property to reproducibly maintain and display part or the whole of the chemical and immunochemical reactivity of their components considered separately. Said conjugate is endowed with the property of reproducibly displaying the reactivity of each ordered n-uple or pair, without lack of generality, of non accessory components when said reactivity is jointly assayed by immunometric methods. The method for the preparation of said bioconjugate comprises:
1] Functionalization, if required, of the species referred to as non accessory components
2] Functionalization, if required, of the species referred to as accessory components
3] Application of a combinatorial methodology for the identification of the best reaction conditions
4] Synthesis of the conjugate in the conditions identified in step 3]

- Step 1 requires the use of suitable reagents for the introduction of groups that are not present or are not sufficiently represented in the structure of the starting biomolecule. As used herein, "non accessory components" means species in which desired functional groups are introduced exploiting methods reported in the literature. As used herein, "functional groups" means any molecular moieties that are not present or are not sufficiently represented in the structure of the starting biomolecule. A non-limiting example of these groups is the following: thiols, pyridyl disulfides, azides, alkynes, alkenes, dienes, biotin, hydrazides hydrazine, aldehydes ketones, phenacyl halides and pseudohalides, primary alkyl halides and pseudohalides, secondary alkyl halides and pseudohalides, tertiary alkyl halides and pseudohalides, aromatic, benzylic, allylic halides and pseudohalides, maleimides, (His)x sequences. Conditions and methods are described in the literature and are known to those skilled in the art. As used herein, "functionalized non accessory components" also means biomolecules, fragments, sequences thereof comprising at least one of the following groups: thiol, amine, carboxy, hydroxyl, imidazole, phenoxy guanidinium, phenyl groups.
- Step 2 requires the use of suitable reagents for the introduction of groups that are not present or are not sufficiently represented in the structure of the starting biomolecule. As used herein "functionalized accessory components" means amino acids polymers or other polymeric species in which functional groups are introduced exploiting methods reported in the literature. As used herein, "functional groups" means any molecular moieties that are not present or are not sufficiently represented in the structure of the starting biomolecule. A non-limiting example of these groups is the following: thiols, pyridyl disulfides, azides, alkynes, alkenes, dienes, biotin, hydrazides hydrazine, aldehydes ketones, phenacyl halides and pseudohalides, primary alkyl halides and pseudohalides, secondary alkyl halides and pseudohalides, tertiary alkyl halides and pseudohalides, aromatic, benzylic, allylic halides and pseudohalides, maleimides, (His)x sequences. Moreover, as used herein "accessory components" comprise homobifunctional cross-linkers. A non limiting example of homobifunctional cross-linkers is the following: active esters of dicarboxylic acids such as: suberic acid bis N-hydroxysuccinimide ester, sebacic acid bis *N*-hydroxysuccinimide ester, 3,3'-dithiodipropionic acid glutaraldehyde, 1,4-Bis[3-(2-pyridyldithio)propionamido]butane, 4,4'-diisothiocyanatestilbene-2,2' disulfonic acid disodium salt hydrate, adipic acid dihydrazide, bis[2-(4-azidosalicylamido)ethyl] disulfide, bis[2-(*N*-succinimidyl-oxycarbonyloxy)ethyl]sulfone, dimethyl 3,3 '-dithioproprionimidate dihydrochloride, dimethyl pimelimidate dihydrochloride, ethylene glycol-bis(succinic acid *N*-hydroxysuccinimide ester), bis(polyethylene glycol bis[imidazolyl carbonyl]).

As used herein "accessory components" also means heterobifunctional crosslinkers. A non limiting example of homobifunctional cross-linkers is the following: active esters of functionalized carboxylic acids such as *N*-hydroxysuccinimide esters of 3-(2-pyridyldithio)propionic acid, 3-maleimidobenzoic acid, 4-(4-maleimidophenyl)butyric acid, 4-(*N-*maleimidomethyl)cyclohexane-1-carboxylic acid, 4-maleimidobutyric acid, 5-azido-2-nitrobenzoic acid, 6-(4-azido-2-nitrophenylamino)hexanoic acid, 6-maleimidohexanoic acid, bromoacetic acid, iodoacetic acid, maleimidoacetic acid, S-acetylthioglycolic acid, maleimidoethyl succinic acid. Other bifunctional compounds such as 4-(*N*-maleimido)benzophenone, 4-azidophenacyl bromide, *N*-(3-dimethylaminopropyl)-*N*'-ethylcarbodiimide, *N-*((2-pyridyldithio)ethyl)-4-azidosalicylamide. As used herein "accessory components" means also proteins such as avidin, streptavidin, neutravidin, lectins, and other proteins endowed with the properties to selectively recognize those groups naturally present or artificially introduced in the structure of the non accessory components.
- Step 3 comprises.
   1) a series of mixing operations using solutions of non accessory components as well as accessory components and buffer solutions. Said mixing operations are carried out in one or more reaction vessels, in the latter case, said reaction vessels are conveniently but not necessarily arranged in ordered arrays. The mixing operations are followed, if required, by one or more purification steps.
   2) one or more immunometric procedures aimed to assess the reactivity of each ordered pair of non accessory components used in the conjugate synthesis.

For what concerns step 1), we consider, as a non limiting example, Q reaction vessels of given volume, conveniently arranged in an ordered array of m row and n columns (Q = m x n), conveniently but not necessarily constituted of a single object of inert material. In a typical example, each reaction compartment is loaded with a known concentration, A₀ of the protein species A. Said species may either be an accessory or non accessory component. The procedure requires a series of t (t = 1, 2, 3,..., k) cycles comprising the delivery and mixing of suitable buffers or protein solutions in suitable buffers. Cycle t = 1 require the following operations non necessarily carried out in this sequence.
a) according to the vertical direction of the reaction array, protein B is serially diluted. Said serial dilution is carried out according to a ratio defined as 2^{j1} (j1 = 0, 1, 2, ···, k where k <n-1) starting from the concentration B₀.
b) According to the horizontal direction of the reaction vessels array, a dilution of the species C is performed. Said species C may be either an accessory or a non accessory component. Said dilutions are performed according to the ratio 2^{il} + hᵢ₁ (i1 = 0, 1, 2,···, k where k < m-1 and hᵢ₁ is conveniently chosen so that |hᵢ₁| < 2^{il}) The initial concentration of species C is meant to be C₀.

To cycle t = 2 correspond the following operations not necessarily carried out in this order:
a) according to the vertical direction of the reaction array, protein D is serially diluted. Said serial dilution is carried out according to a ratio defined as 2^{j2} (j1 = 0, 1, 2, ···, k where k <n-1) starting from the concentration D₀₃.
b) according to the horizontal direction of the reaction vessels array, a dilution of the species E is performed. Said species E may be either an accessory or a non accessory component. Said dilutions are performed according to the ratio 2ⁱ² + hᵢ₂ (i2 = 0, 1, 2,···, k where k < m-1 and hᵢ₂ is conveniently chosen so that |hᵢ₂| < 2ⁱ²) The initial concentration of species E is meant to be E₀.

The process is continued until all the t cycles necessary to the delivery of all the protein solutions and buffers are completed. At the end of the process, in each reaction compartment qᵢⱼ the concentrations of all the species considered is the following: [δA₀, δB₀*2^{-j1}, δC₀*(2ⁱ¹+hᵢ₁)⁻¹, δD₀*2^{-j2}, δE₀*(2ⁱ²+hᵢ₂)⁻¹, ····], being δ the dilution factor.

At the end of the process, the content of each reaction compartment is assayed for its reactivity using immunometric methods.

For what concerns point 2) the selection of the best conditions require:
a) Immobilization, on plastic microtiter plates for ELISA determination, of monoclonal or polyclonal antibodies or Fab or F(ab')₂ fragments thereof directed against one of the non accessory components comprised in the conjugate.
b) Incubation of the samples containing the conjugate of interest with the immobilized antibodies or fragments thereof.
c) Washing of the complex formed between the conjugate and the immobilized antibody or fragment thereof.
d) Detection of the presence of the antibody-conjugate complex with the aid of reagents selective or specific for one of the non accessory components different from that involved in the interaction between the conjugate and the immobilized antibody or fragment thereof. Identification of the reaction compartments containing conjugates with the desired reactivity and identification of the reaction parameters t, jt, it, hᵢₜ δ, A₀, B₀, ····, Z₀ corresponding to those qᵢⱼ compartment containing the conjugates displaying the desired reactivity.

All these methods are known by those skilled in the art. -Step 4 require the synthesis of the conjugate identified during the phase 3.

The preferred method for the preparation of the conjugate comprises:
1] functionalization by biotinylation of the species collectively described as non accessory components
2] use of avidin, streptavidin or neutravidin as accessory component.
3]application of a combinatorial methodology for the determination of the best reaction conditions for the obtainment of conjugates with the desired reactivity.
4] synthesis of the conjugates according to the conditions identified in said combinatorial screening.

The present invention will be described by some non-limiting examples:

### EXAMPLE 1: conjugate displaying the reactivity of IgM and SCCA

SCCA and IgM were biotinylated using biotinamidohexanoyl-6-aminohexanoic acid *N*-hydroxysuccinimide ester (Sigma-Aldrich B3295-50MG) and extensively dialyzed against PBS. The chemistry and the conditions used in the biotinylation process are known to those skilled in the art. A microtiter plate for ELISA determination (Greiner, Medium binding STRIP PLATE) was treated with a 5% solution of dried skimmed milk in PBS (PBS-M) (300 µl/well). After 12 hours of incubation at 4°C the plate was washed with PBS containing 0.05% Tween 20 (PBS-T). Each well was loaded with the following accessory and non accessory components:
a) Avidin (Applichem) 0-20 µg in PBS
b) Biotinylated IgM, 2.5-40 µg, preferably 10 µg, biotinylated SCCA with concentration comprised in the range 20-0.05 µg/ml.

The plate was allowed to stand at room temperature for 1 hour. After the incubation time, the content of each well was tested to assess the reactivity due to the conjugate. An ELISA microtiter plate (Greiner 762071, High binding STRIP PLATE) was treated with 100 µl/well of polyclonal rabbit anti-SCCA antibody (Xeptagen) at the final concentration of 10 µg/ml and incubated at 4°C in a humid chamber for 12 hours. The plate was washed 3 times with 300 µl/well of PBS containing 0.05% Tween 20 (PBS-T) (Sigma-Aldrich P-1379), each well was filled with 300 µl of PBS containing 3% bovine serum albumin (Sigma-Aldrich A4503-1KG) and incubated at room temperature for 2 hours in a humid closed chamber. The plate was washed 3 times with PBS-T (300 µl/well), in each well 100 µl of the conjugate solutions were loaded and the plate was incubated for 1 hour at room temperature. The plate was washed with PBS-T buffer 6 x 300 µl/well and each well was loaded with 100 µl of anti-human IgM-HRP conjugated antibody (Sigma-Aldrich A0420-1ML) diluted 1000 times, the plate was incubated for 1 hour. The plate was washed with PBS-T buffer 6 x 300 µl/well and each well was loaded with 150 µl of ABTS [2,2'-azino-bis(3-ethylbenzthiazoline-6-sulphonic acid)] 0.2 mg/ml in phosphate-citrate buffer pH 5.0 containing 5 mM hydrogen peroxide. The plate was incubated at 37±2 °C for 20 minutes, the optical density was measured at 405 nm with a suitable microtiter plate reader. The conditions corresponding to the most reactive preparations were used for the preparative scale synthesis of the conjugate.

### EXAMPLE 2: conjugate displaying the reactivity of the CEA-IgM immunocomplex

CEA and IgM were biotinylated using biotinamidohexanoyl-6-aminohexanoic acid *N*-hydroxysuccinimide ester (Sigma-Aldrich B3295-50MG) and extensively dialyzed against PBS. The chemistry and the conditions used in the biotinylation process are known to those skilled in the art. A microtiter plate for ELISA determination (Greiner, Medium binding STRIP PLATE) was treated with a 5% solution of dried skimmed milk in PBS (PBS-M) (300 µl/well). After 12 hours the plate was washed with PBS containing 0.05% Tween 20 (PBS-T). Each well was loaded with the following accessory and non accessory components:
a) Biotinylated IgM, 2.5-40 µg, preferably 10 µg, dissolved in PBS buffer
b) Biotinylated human CEA with concentration comprised in the range 20-0.05 µg/ml in PBS, Avidin (Applichem) in the range 0-20 µg/well in PBS.

The plate was allowed to stand at room temperature for 1 hour. After the incubation time, the content of each well was tested to assess the reactivity due to the conjugate. An ELISA microtiter plate (Greiner 762071, High binding STRIP PLATE) was treated with 100 µl/well of polyclonal rabbit anti-CEA antibody (Dako A0115) at the final concentration of 10 µg/ml and incubated at 4°C in a humid chamber for 12 hours. The plate was washed 3 times with 300 µl/well of PBS containing 0.05% Tween 20 (PBS-T) (Sigma-Aldrich P-1379), each well was filled with 300 µl of PBS containing 3% bovine serum albumin (Sigma-Aldrich A4503-1KG) and incubated at room temperature for 2 hours in a humid closed chamber. The plate was washed 3 times with PBS-T (300 µl/well), in each well 100 µl of the conjugate solutions were loaded and the plate was incubated for 1 hour at room temperature. The plate was washed with PBS-T buffer 6 x 300 µl/well and each well was loaded with 100 µl of a 10 µl/ml solution of anti-human IgM-HRP conjugated antibody (Sigma-Aldrich A0420-1ML) diluted 1000 times, the plate was incubated for 1 hour. The plate was washed with PBS-T buffer 6 x 300 µl/well and each well was loaded with 150 µl of ABTS [2,2'-azino-bis(3-ethylbenzthiazoline-6-sulphonic acid)] 0.2 mg/ml in phosphate-citrate buffer pH 5.0 containing 5 mM hydrogen peroxide. The plate was incubated at 37±2 °C for 20 minutes, the optical density was measured at 405 nm with a suitable microtiter plate reader. The conditions corresponding to the most reactive preparations were used for the preparative scale synthesis of the conjugate.

### EXAMPLE 3: conjugate displaying the reactivity of the ordered pairs of proteins (SCCA-IgM) (CEA-IgM), (SCCA-CEA)

CEA, SCCA and IgM were biotinylated using biotinamidohexanoyl-6-aminohexanoic acid *N*-hydroxysuccinimide ester (Sigma-Aldrich B3295-50MG) and extensively dialyzed against PBS. The chemistry and the conditions used in the biotinylation process are known to those skilled in the art. A microtiter plate for ELISA determination (Greiner, Medium binding STRIP PLATE) was treated with a 5% solution of dried skimmed milk in PBS (PBS-M) (300 µl/well). After 12 hours the plate was washed with PBS containing 0.05% Tween 20 (PBS-T). Each well was loaded with the following accessory and non accessory components:
a) Avidin (Applichem) 0-20 µg in PBS
b) Biotinylated IgM, 2.5-40 µg, preferably 10 µg, biotinylated SCCA concentration comprised in the range 20-0.05 µg/ml, biotinylated CEA in concentration comprised in the range 20-0.05 µg/ml in PBS. The plate was allowed to stand at room temperature for 1 hour. After the incubation time, the content of each well was tested to assess the reactivity due to the conjugate.

### Screening for the reactivity of the ordered pair CEA-IgM

A microtiter ELISA plate (Greiner 762071, High binding STRIP PLATE) was treated with 100 µl/well of polyclonal rabbit anti-CEA antibody (Dako A0115) at the final concentration of 10 µg/ml and incubated at 4°C in a humid chamber for 12 hours. The plate was washed 3 times with 300 µl/well of PBS containing 0.05% Tween 20 (PBS-T) (Sigma-Aldrich P-1379), each well was filled with 300 µl of PBS containing 3% bovine serum albumin (Sigma-Aldrich A4503-1KG) and incubated at room temperature for 2 hours in a humid closed chamber. The plate was washed 3 times with PBS-T (300 µl/well), in each well 100 µl of the conjugate solutions were loaded and the plate was incubated for 1 hour at room temperature. The plate was washed with PBS-T buffer 6 x 300 µl/well and each well was loaded with 100 µl of anti-human IgM-HRP conjugated antibody solution (Sigma-Aldrich A0420-1ML) diluted 1000 times, the plate was incubated for 1 hour. The plate was washed with PBS-T buffer 6 x 300 µl/well and each well was loaded with 150 µl of ABTS [2,2'-azino-bis(3-ethylbenzthiazoline-6-sulphonic acid)] 0.2 mg/ml in phosphate-citrate buffer pH 5.0 containing 5 mM hydrogen peroxide. The plate was incubated at 37±2 °C for 20 minutes, the optical density was measured at 405 nm with a suitable microtiter plate reader.

### Screening for the reactivity of the ordered pair SCCA-IgM

A second ELISA microtiter plate (Greiner 762071, High binding STRIP PLATE) was treated with 100 µl/well of polyclonal rabbit anti-SCCA antibody (Xeptagen) at the final concentration of 10 µg/ml and incubated at 4°C in a humid chamber for 12 hours. The plate was washed 3 times with 300 µl/well of PBS containing 0.05% Tween 20 (PBS-T) (Sigma-Aldrich P-1379), each well was filled with 300 µl of PBS containing 3% bovine serum albumin (Sigma-Aldrich A4503-1KG) and incubated at room temperature for 2 hours in a humid closed chamber. The plate was washed 3 times with PBS-T (300 µl/well), in each well 100 µl of the conjugate solutions were loaded and the plate was incubated for 1 hour at room temperature. The plate was washed with PBS-T buffer 6 x 300 µl/well and each well was loaded with 100 µl of solution of anti-human IgM-HRP conjugated antibody (Sigma-Aldrich A0420-1ML) diluted 1000 times, the plate was incubated for 1 hour. The plate was washed with PBS-T buffer 6 x 300 µl/well and each well was loaded with 150 µl of ABTS [2,2'-azino-bis(3-ethylbenzthiazoline-6-sulphonic acid)] 0.2 mg/ml in phosphate-citrate buffer pH 5.0 containing 5 mM hydrogen peroxide. The plate was incubated at 37±2 °C for 20 minutes, the optical density was measured at 405 nm with a suitable microtiter plate reader.

### Screening for the reactivity of the ordered pair SCCA-CEA

A third microtiter plate for ELISA determinations (Greiner 762071, High binding STRIP PLATE) was treated with 100 µl/well of monoclonal mouse anti-SCCA antibody (Xeptagen) at the final concentration of 10 µg/ml and incubated at 4°C in a humid chamber for 12 hours. The plate was washed 3 times with 300 µl/well of PBS containing 0.05% Tween 20 (PBS-T) (Sigma-Aldrich P-1379), each well was filled with 300 µl of PBS containing 3% bovine serum albumin (Sigma-Aldrich A4503-1KG) and incubated at room temperature for 2 hours in a humid closed chamber. The plate was washed 3 times with PBS-T (300 µl/well), in each well 100 µl of conjugate solutions were loaded and the plates incubated for 1 hour at room temperature. The plate was washed with PBS-T buffer 6 x 300 µl/well and each well was loaded with 100 µl of a 2 µg/ml solution of polyclonal rabbit anti-CEA antibody (Dako A0115) in PBS-T containing 1% BSA, the plate was incubated for 1 hour. The plate was washed with PBS-T buffer 6 x 300 µl/well and each well was loaded with 100 µl of a solution of goat anti-rabbit IgG-HRP conjugated antibody (KPL 474-1506), the plate was incubated for 1 hour. The plate was washed with PBS-T buffer 6 x 300 µl/well and each well was filled with 150 µl of ABTS [2,2'-azino-bis(3-ethylbenzthiazoline-6-sulphonic acid)] 0.2 mg/ml in phosphate-citrate buffer pH 5.0 containing 5 mM hydrogen peroxide. The plate was incubated at 37±2 °C for 20 minutes, the optical density was measured at 405 nm with a suitable microtiter plate reader.

### EXAMPLE 4: conjugate displaying the reactivity of the AFP-IgM immunocomplex

AFP and IgM were biotinylated using biotinamidohexanoyl-6-aminohexanoic acid *N*-hydroxysuccinimide ester (Sigma-Aldrich B3295-50MG) and extensively dialyzed against PBS. The chemistry and the conditions used in the biotinylation process are known to those skilled in the art. A microtiter plate for ELISA determination (Greiner, Medium binding STRIP PLATE) was treated with a 5% solution of dried skimmed milk in PBS (PBS-M) (300 µl/well). After 12 hours the plate was washed with PBS containing 0.05% Tween 20 (PBS-T). Each well was loaded with the following accessory and non accessory components:
a) Avidin (Applichem) 0-20 µg in PBS
b) Biotinylated IgM, 2.5-40 µg, preferably 10 µg, biotinylated AFP with concentration comprised in the range 20-0.05 µg/ml.

The plate was allowed to stand at room temperature for 1 hour. After the incubation time, the content of each well was tested to assess the reactivity due to the conjugate. An ELISA microtiter plate (Greiner 762071, High binding STRIP PLATE) was treated with 100 µl/well of polyclonal rabbit anti-AFP antibody (Dako A008) at the final concentration of 10 µg/ml and incubated at 4°C in a humid chamber for 12 hours. The plate was washed 3 times with 300 µl/well of PBS containing 0.05% Tween 20 (PBS-T) (Sigma-Aldrich P-1379), each well was filled with 300 µl of PBS containing 3% bovine serum albumin (Sigma-Aldrich A4503-1KG) and incubated at room temperature for 2 hours in a humid closed chamber. The plate was washed 3 times with PBS-T (300 µl/well), in each well 100 µl of the conjugate solutions were loaded and the plate incubated for 1 hour at room temperature. The plate was washed with PBS-T buffer 6 x 300 µl/well and each well was loaded with 100 µl of anti-human IgM-HRP conjugated antibody solution (Sigma-Aldrich A0420-1ML) diluted 1000 times, the plate was incubated for 1 hour. The plate was washed with PBS-T buffer 6 x 300 µl/well and each well was loaded with 150 µl of ABTS [2,2'-azino-bis(3-ethylbenzthiazoline-6-sulphonic acid)] 0.2 mg/ml in phosphate-citrate buffer pH 5.0 containing 5 mM hydrogen peroxide. The plate was incubated at 37±2 °C for 20 minutes, the optical density was measured at 405 nm with a suitable microtiter plate reader. The conditions corresponding to the most reactive preparations were used for the preparative scale synthesis of the conjugate.

### EXAMPLE 5: quantitative determination of the specific ICC containing the protein SCCA1 and/or its variants and the autoantibody specific to them in serum from Hepatocellular carcinoma (HCC) using as calibrator the object of the following invention.

A microplate for ELISA determinations (Greiner 762071, High binding STRIP PLATE) was treated with 100 µl of a solution of rabbit polyclonal anti-SCCA antibody (Xeptagen) in PBS buffer for 12 hours at 4°C. The plate was washed with PBS containing 0.05% Tween 20. To each well were added 300 µl of a 1% solution of bovine serum albumin in PBS (BSA, Sigma-Aldrich, A-4503) (PBS-1% BSA). After a 2 hours incubation at room temperature, the plate was washed 6 times with PBS-T, and filled with 100 µl of human serum at different dilutions. In the same plate, a series of wells was used to construct the calibration curve for the specific CIC SCCA-IgM using the calibrator prepared according to the example 1. The concentration range spanned was between 1000-15 arbitrary units per milliliter (AU/ml). The plate was incubated for 1 hour at room temperature. The plate was washed 6 times with a solution of PBS-T and filled with 100 µl of a solution of polyclonal antibody, directed against human immunoglobulins, conjugated to horseradish peroxidase diluted 1000 times and incubated for 1 hour at room temperature. At the end of the incubation time, the plate was washed 6 times with a solution of PBS-T and filled with 200 µl of a solution of chromogenic substrate ABTS [2,2'-azino-bis(3-ethylbenzthiazoline-6-sulphonic acid)] 0.2 mg/ml in phosphate-citrate buffer pH 5.0 containing 5 mM hydrogen peroxide. After incubation at various times (20-30 minutes) at 37 °C, the absorbance at 405 nm was read using a suitable plate reader (Biorad). The concentration of SCCA-IgM in the samples were determined by interpolation of the absorbance values on the standard curve. The analysis of sera from HCC patients according to the previously described method confirmed the presence of the specific CIC, while the control sera obtained from healthy subjects resulted to be negative. (100% specificity).

Hence, with reference to the above discussion and the attached table, what we claim is:

## Claims

1. A method for optimizing the synthesis of an immuno-complex/immunoconjugate molecule on a substrate comprising the following:
a) step of adding a linker to one or more immunoglobulins such as IgA, IgG, IgM, IgE, IgD,
b) step of adding a linker to one or more antigens, wherein said antigens are tumor markers,
c) step of binding molecules produced in steps a) and b) together by mixing with a conjugation, cross linker, scaffold or accessory component,
d) bind conjugate produced in step c) to a surface of a separate well using an immobilized anti antigen antibody, CEA or Squamous Cell Carcinoma Antigen (SCCA) or AFP, and assay the binding reaction with a labeled anti IgM antibody in a sandwich assay,
e) repeat the above steps in an array, i.e. in parallel, with different reactant concentrations in order to identify best concentration for producing conjugates using the ELISA sandwich assay results,
f) synthesizing conjugates according to the concentration used in the best ELISA result,

2. Method of claim 1, **characterized by** the fact that said linker of steps a) and b) is biotin.

3. Method of claim 1, **characterized by** the fact that said cross linker of step c) is avidin, streptavidin or neutravidin.

4. Method of claim 1, **characterized by** the fact that said antigen is one of the following tumor markers:
▪ Ki-67 for Astrocytoma;
▪ Fibronectin, Hepatoma up regulated protein (HURP), Mucin 7 (MUC7), NMP22, Prostate stem cell antigen (PSCA), Telomerase, Tissue polypeptide antigen (TPA) for bladder cancer;
▪ CA 15-3, CA 27.29, AFP, CEA, CA 15-3, Ceruloplasmin, TPA, Cytokeratin 19 (CK19), Maspin, c-Met Cytochrome P450 3A4, Epithelial glycoprotein 2 (EGP2), ErbB-2 Her2/Neu MMP-9 for breast cancer;
▪ Human kallikrein 5 (hK5) for breast and ovarian cancer;
▪ NMP179 for the cervical squamous epithelium cancer;
▪ CEA, Cytokeratin 19 (CK19), Cytokeratin 20 (CK20), Cytokeratin, Guanylyl cyclase C (GCC), Her2/Neu, MUC6, MUC5AC, RelA, NF-kb, for colorectal cancer;
▪ Bcl-6, CD10, for B-cells lymphoma;
▪ CEA, for endometrial carcinoma;
▪ Cystein-rich fibroblast growth factor receptor 1 (CFR-1)m p27, MIB-1 for stomach cancer;
▪ Chromogranin A (CgA), Neuron specific enolase (NSE), Synaptophysin, Leu-7, beta III-tubulin for gastrointestinal carcinoma;
▪ Cytokeratin, Epithelial membrane antigen (EMA), Hyaluronidase (HYAL1), Latent membrane protein 1 (LMP-1) for head and neck carcinoma;
▪ Alpha-fetoprotein (AFP), Des-gamma-carboxy prothrombin (DCP), Squamous Cell Carcinoma Antigen (SCCA) for hepatocellular carcinoma;
▪ Cellular retinol binding protein 1 (CRBP1), Glypcan-3, Telomerase for hepatocellular carcinoma;
▪ Preferentially expressed antigen of melanoma (PRAME) for leukemia and multiple myeloma;
▪ CEA, Chromogranin A, NSE, Vascular endothelial growth factor (VEGF), Stem cell factor (SCF), Hepatocyte growth factor/Scatter factor (HGF/SF) for lung cancer;
▪ Epidermal growth factor receptor (EGFR), M2-PK, CYFRA 21-1, NSE, SCC, for lung cancer;
▪ Neuron specific enolase (NSE), S-100B, Tyrosinase, LDH, for melanoma;
▪ Cytokeratin 20 (CK20), Prostate stem cell antigen (PSCA) for gastrointestinal cancer;
▪ Medkine (MK) for gastric carcinoma;
▪ CA 125, CASA, for ovarian cancer;
▪ CA 19-9 for pancreatic carcinoma;
▪ Survivin, p53, Bcl-2 for pancreatic carcinoma;
▪ Secretogranin II-derived peptide EM66 for pheochromocytoma;
▪ GLUT1, GLUT12, PSA, Insulin-like growth factor binding protein 2 (IGBFB2) for prostate cancer;
▪ Myogenin, MyoD1 for rhabdomyosarcoma;
▪ Dipeptidyl Peptidase IV (DPP IV/CD 26) for thyroid cancer (papillary carcinoma);
▪ Peroxisome proliferating activated receptor gamma (PPAR gamma) for thyroid cancer (papillary carcinoma).

## Patentansprüche

1. Ein Verfahren zur Optimierung der Synthese eines Immunkomplex-/Immunkonjugatmoleküls auf einem Substrat, das Folgendes umfasst:
a) Schritt des Zusatzes eines Linkers zu einem oder mehreren Immunoglobulinen wie IgA, IgG, IgM, IgE, IgD,
b) Schritt des Zusatzes eines Linkers zu einem oder mehreren Antigenen, wobei die besagten Antigene Tumormarker sind,
c) Schritt der Bindung der in den Schritten a) und b) gebildeten Moleküle durch Mischung mit einer Konjugation, einem Vernetzungsmittel, einem Trägersystem bzw. einer Hilfskomponente,
d) Bindung des in Schritt c) erzeugten Konjugats an eine Oberfläche eines separaten Wells mittels eines immobilisierten Anti-Antigen-Antikörpers, CEA oder Squamous Cell Carcinoma Antigen (SCCA) oder AFP, und Nachweis der Bindungsreaktion mit einem markierten Anti-IgM-Antikörper in einem Sandwich-Assay,
e) Wiederholung der obigen Schritte an einem Array, d.h. parallel, mit unterschiedlichen Reaktandkonzentrationen, um die besten Konzentrationen für die Herstellung von Konjugaten unter Nutzung der Resultate des ELISA Sandwich-Assays zu identifizieren,
f) Synthetisierung der Konjugate entsprechend der im besten ELISA-Ergebnis verwendeten Konzentration.

2. Verfahren nach Anspruch 1, **dadurch gekennzeichnet, dass** der besagte Linker aus Schritt a) und b) Biotin ist.

3. Verfahren nach Anspruch 1, **dadurch gekennzeichnet, dass** das besagte Vernetzungsmittel aus Schritt c) Avidin, Streptavidin oder NeutrAvidin ist.

4. Verfahren nach Anspruch 1, **dadurch gekennzeichnet, dass** das besagte Antigen eines der nachstehenden Tumormarker ist:
▪ Ki-67 für Astrozytome;
▪ Fibronektin, Hepatoma-upregulated protein (HURP), Mucin 7 (MUC7), NMP22, Prostata-Stammzellen-Antigen (PSCA), Telomerase, Tissue Polypeptide Antigen (TPA) für Blasenkrebs;
▪ CA 15-3, CA 27.29, AFP, CEA, CA 15-3, Caeruloplasmin, TPA, Cytokeratin 19 (CK19), Maspin, c-Met Cytochrome P450 3A4, Epithelial glycoprotein 2 (EGP2), ErbB-2 Her2/Neu MMP-9 für Brustkrebs;
▪ Kallikrein 5 (hK5) für Brust- und Eierstockkrebs;
▪ NMP179 für das zervikale Plattenepithelkarzinom;
▪ CEA, Cytokeratin 19 (CK19), Cytokeratin 20 (CK20), Cytokeratin, Guanylylcyclase C (GCC), Her2/Neu, MUC6, MUC5AC, RelA, NF-kb, für kolorektales Karzinom;
▪ Bcl-6, CD 10, für B-Zellen-Lymphom;
▪ CEA für Endometriumkarzinom;
▪ Cysteinreicher Fibroblastenwachstumsfaktor-Rezeptor 1 (CFR-1) m p27, MIB-1 für Magenkrebs;
▪ Chromogranin A (CgA), Neuronenspezifische Enolase (NSE), Synaptophysin, Leu-7, Beta Ill-Tubulin für Magendarmkrebs;
▪ Cytokeratin, Epitheliales Membranantigen (EMA), Hyaluronidase (HYAL1), Latentes Membranprotein 1 (LMP-1) für Kopf- und Halskrebs;
▪ Alpha-Fetoprotein (AFP), des-Gamma-Carboxy Prothrombin (DCP), Squamous Cell Carcinoma Antigen (SCCA) für Leberzellkarzinom;
▪ Cellular retinol binding protein 1 (CRBP1), Glypcan-3, Telomerase für Leberzellkarzinom;
▪ Preferentially expressed antigen of melanoma (PRAME) für Leukämie und Multiples Myelom;
▪ CEA, Chromogranin A, NSE, Vascular endothelial growth factor (VEGF), Stammzellenfaktor (SCF), Hepatozyten-Wachstumsfaktor/Scatter factor (HGF/SF) für Lungenkrebs;
▪ Epidermal Growth Factor Receptor (EGFR), M2-PK, CYFRA 21-1, NSE, SCC für Lungenkrebs;
▪ Neuronenspezifische Enolase (NSE), S-100B, Tyrosinase, LDH für Melanom;
▪ Cytokeratin 20 (CK20), Prostata-Stammzellen-Antigen (PSCA) für Magendarmkrebs;
▪ Medkine (MR) für Magenkrebs;
▪ CA 125, CASA für Eierstockkrebs;
▪ CA 19-9 für Pankreaskarzinom;
▪ Survivin, p53, Bcl-2 für Pankreaskarzinom;
▪ Secretogranin II-derived peptide EM66 für Phäochromozytom;
▪ GLUT1, GLUT 12, PSA, Insulin-like growth factor binding protein 2 (IGBFB2) für Prostatakrebs;
▪ Myogenin, MyoDl für Rhabdomyosarkom;
▪ Dipeptidyl Peptidase IV (DPP IV/CD 26) für Schilddrüsenkrebs (papilläres Karzinom);
▪ Peroxisome proliferating activated receptor gamma (PPAR gamma) für Schilddrüsenkarzinom (papilläres Karzinom).

## Revendications

1. Méthode pour l'optimisation de la synthèse d'une molécule immunocomplexe/immunoconjuguée sur un substrat comprenant ce qui suit :
a) une phase qui consiste à ajouter un liant à une ou plusieurs immunoglobulines telles que IgA, IgG, IgM, IgE, IgD,
b) une phase qui consiste à ajouter un liant à un ou plusieurs antigènes, où lesdits antigènes sont des marqueurs tumoraux,
c) une phase qui consiste à lier ensemble des molécules produites dans les phases a) et b) en les mélangeant avec une conjugaison, un agent de réticulation, un échafaudage ou un élément accessoire,
d) une liaison d'un conjugué produit dans la phase c) avec une surface d'un puits séparé en utilisant un anticorps anti antigène immobilisé, CEA ou antigène du carcinome de cellules squameuses (SCCA) ou AFP, et essayer la réaction de liaison avec un anticorps anti IgM étiqueté dans un test en sandwich,
e) une répétition des phases indiquées ci-dessus dans un test, par ex. en parallèle, avec des concentrations du réactif différentes de façon à identifier la meilleure concentration pour produire des conjugués en utilisant les résultats du test ELISA en sandwich,
f) synthétiser des conjugués selon la concentration utilisée dans le meilleur résultat ELISA.

2. Méthode selon la revendication 1, **caractérisée en ce que** ledit liant des phases a) et b) est biotine.

3. Méthode selon la revendication 1, **caractérisée en ce que** ledit agent de réticulation de la phase c) est avidine, streptavidine ou neutravidine.

4. Méthode selon la revendication 1, **caractérisée en ce que** ledit antigène est un des marqueurs tumoraux suivants :
• Ki-67 pour l'Astrocytome ;
• Fibronectine, Hepatome upregulated protein (HURP), Mucine 7 (MUC7), NMP22, NMP22, antigène de cellule souche de la prostate (PSCA), Télomérase, antigène tissulaire polypeptidique (TPA) pour le cancer de la vessie ;
• CA 15-3, CA 27.29, AFP, CEA, CA 15-3, Céruloplasmine, TPA, CEA, Cytokératine 19 (CK19), Maspine, c-Met Cytochrome P450 3A4, glycoprotéine épithélial 2 (EGP2), Cytokératine 19 (Ck 19), ErbB-2 Her2/Neu MMP-9 pour le cancer du sein ;
• Kallicréine humaine 5 (hK5) pour le cancer du sein et des ovaires ;
• NMP179 pour le cancer de l'épithélium squameux du col de l'utérus ;
• CEA, Cytokératine 19 (CK19), Cytokératine 20 (CK20), Cytokératine, Cytokératine 20 (CK20), CEA, Guanylyl cyclase C (GCC), Her2/Neu, MUC6, MUC5AC, RelA, NF-kb, pour le cancer colorectal ;
• Bcl-6, CD10, pour le lymphome à cellules B ;
• CEA, pour le cancer endométrial ;
• Récepteur du facteur de croissance des fibroblastes riches en cystéine 1 (CFR-1)m p27, MIB-1 pour le cancer de l'estomac ;
• Chromogranine A (CgA), Énolase spécifique des neurones (ESN), Synaptophysine, Leu-7, béta III-tubuline pour le carcinome grastro-intestinal ;
• Cytokératine, antigène épithélial membranaire (EMA), Hyaluronidase (HYAL1), protéines latentes de la membrane 1 (LMP-1) pour le carcinome de la tête et du cou ;
• Alpha-foetoprotéine (AFP), Des-gamma-carboxy prothrombine (DCP), antigène du carcinome des cellules squameuses (SCCA) pour le carcinome hépatocellulaire ;
• Protéine cellulaire de liaison au rétinol 1 (CRBP1), Glypcan-3 , Télomérase pour le carcinome hépatocellulaire ;
• Antigène préférentiellement exprimé dans le mélanome (PRAME) pour la leucémie et le myélome multiple ;
• CEA, Chromogranine A, NSE, Facteur de croissance de l'endothélium vasculaire (VEGF), Stem cell factor [*Facteur de croissance des cellules souches*] (SCF), Facteur de croissance hépatocyte/Scatter factor (HGF/SF) pour le cancer du poumon ;
• Récepteur du facteur de croissance épidermique (EGFR), M2-PK, CYFRA 21-1, NSE, SCC, pour le cancer du poumon ;
• Énolase spécifique des neurones (ESN), S-100B, Tyrosinase, LDH, pour le mélanome ;
• Cytokératine 20 (CK20), Antigène de cellule souche de la prostate (PSCA) pour le cancer gastro-intestinal ;
• Medkine (MK) pour le carcinome de l'estomac ;
• CA 125, CASA, CA 125 pour le cancer des ovaires ;
• CA 19-9 pour le carcinome pancréatique ;
• Survivine, p53, Bcl-2 pour le carcinome pancréatique ;
• Secretogranine II-peptide dérivé EM66 pour le phéochromocytome ;
• GLUT1, GLUT12, Protéine de liaison de facteur de croissance de type insuline 2 (IGBFB2) pour le cancer de la prostate ;
• Myogenine, MyoD1 pour le rhabdomyosarcome ;
• Dipeptidyl Peptidase IV (DPP IV/CD 26) pour le cancer de la thyroïde (carcinome papillaire) ;
• Récepteurs activables par les proliférateurs des peroxysomes gamma (PPAR gamma) pour le cancer de la thyroïde (carcinome papillaire).
